# EUROPEAN PATENT SPECIFICATION

(11) **EP 3 744 323 B1**
(45) Date of publication and mention of the grant of the patent: **02.12.2026**
(21) Application number: 19743131.5
(22) Date of filing: 25.01.2019
(51) Int. Cl.: A61K 31/202, A23L 33/115, A23L 33/175, A61K 31/198, A61P 25/04, A61P 43/00, A61P 25/02

(54) **COMPOSITION COMPRISING AN N-3 FAT ACID AND L-SERINE FOR USE IN PREVENTING OR IMPROVING NOCICEPTIVE PAIN**
ZUSAMMENSETZUNG MIT EINER N-3-FETTSÄURE UND L-SERIN ZUR VERWENDUNG BEI DER VORBEUGUNG ODER VERBESSERUNG VON NOZIZEPTIVEM SCHMERZ
COMPOSITION COMPRENANT UN ACIDE GRAS N-3 ET DE LA L-SÉRINE POUR UNE UTILISATION DANS LA PRÉVENTION OU L'AMÉLIORATION DE LA DOULEUR NOCICEPTIVE

(30) Priority: 26.01.2018 JP 2018011964
(43) Date of publication of application: 02.12.2020
(73) Proprietor: AJINOMOTO CO., INC., Chuo-ku Tokyo 104-8315 (JP)
(72) Inventor: SUGA, Yasuyo, Kawasaki-shi, Kanagawa 210-8681 (JP); SASAHARA, Ikuko, Kawasaki-shi Kanagawa 210-8681 (JP); HASHIMOTO, Masaki, Tokyo 104-8315 (JP); MINE, Tomoyuki, Tokyo 104-8315 (JP); TAKADA, Michihiro, Kawasaki-shi Kanagawa 210-8681 (JP); ISHII, Makoto, Kawasaki-shi Kanagawa 210-8681 (JP); KANZAKI, Michiya, Tokyo 104-8315 (JP)
(74) Representative: Mewburn Ellis LLP
(86) International application number: PCT/JP2019/002387
(87) International publication number: WO 2019/146735

(56) References cited:
- WO-A1-2015/142702
- WO-A1-2015/163316
- WO-A1-2016/152054
- JP-A- 2005 529 980
- JP-A- 2015 091 817
- US-A1- 2017 035 721
- NOBRE MARIA ELIZABETH PEREIRA ET AL: "Eicosapentaenoic acid and docosahexaenoic acid exert anti-inflammatory and antinociceptive effects in rodents at low doses", NUTRITION RESEARCH, ELSEVIER, AMSTERDAM, NL, vol. 33, no. 5, 3 April 2013 (2013-04-03), pages 422 - 433, XP028563943, ISSN: 0271-5317, DOI: 10.1016/J.NUTRES.2013.02.011
- YOSHIKAWA MASANOBU ET AL: "Activation of supraspinal NMDA receptors by both D-serine alone or in combination with morphine leads to the potentiation of antinociception in tail-flick test of rats", EUROPEAN JOURNAL OF PHARMACOLOGY, vol. 565, no. 1-3, 1 June 2007 (2007-06-01), NL, pages 89 - 97, XP055862293, ISSN: 0014-2999, DOI: 10.1016/j.ejphar.2007.02.042
- KIYA T ET AL: "Role of satellite cell-derived l-serine in the dorsal root ganglion in paclitaxel-induced painful peripheral neuropathy", NEUROSCIENCE, NEW YORK, NY, US, vol. 174, 3 February 2011 (2011-02-03), pages 190 - 199, XP027589595, ISSN: 0306-4522, [retrieved on 20110108]
- HIYAMA AKIHIKO ET AL: "Evaluation of quality of life and neuropathic pain in patients with low back pain using the Japanese Orthopedic Association Back Pain Evaluation Questionnaire", EUROPEAN SPINE JOURNAL, SPRINGER BERLIN HEIDELBERG, BERLIN/HEIDELBERG, vol. 24, no. 3, 14 December 2014 (2014-12-14), pages 503 - 512, XP035473341, ISSN: 0940-6719, [retrieved on 20141214], DOI: 10.1007/S00586-014-3723-Y
- JEONG, Y. C. ET AL.: "Inhibition of mitogen- activated protein kinases phosphorylation plays an important role in the anti-nociceptive effect of pregabalin in zymosan-induced inflammatory pain model", BIOLOGICAL AND PHARMACEUTICAL BULLETIN, vol. 37, no. 10, 2014, pages 1694 - 1698, XP055628820, ISSN: 0918-6158
- GUERRERO, A. T. G. ET AL.: "Toll-like receptor 2/MyD88 signaling mediates zymosan-induced joint hypernociception in mice: Participation of TNF-a, IL -Ibeta and CXCL/KC", EUROPEAN JOURNAL OF PHARMACOLOGY, vol. 674, no. 1, 2011, pages 51 - 57, XP028126047, ISSN: 0014-2999
- ALVES, S. M. ET AL.: "Anti-inflammatory and anti- nociceptive effects of strontium ranelate on the zymosan-induced temporomandibular joint inflammatory hypernociception in rats depend on TNF-oi inhibition", PHARMACOLOGICAL REPORTS, vol. 69, no. 4, 2017, pages 764 - 772, XP055628823, ISSN: 1734-1140
- ITO, MIHO ET AL.: "Antinociceptive effect of intracerebroventricular administration or D-serine on formalin-induced pain", JOURNAL OF ANESTHESIA, vol. 28, no. 2, 2014, pages 228 - 234, XP055628824, ISSN: 0913-8668
- ITO, KENJI ET AL.: "Midazolam attenuated the antinociceptive effect of D-serine and morphine at supraspinal level", JOURNAL OF PHARMACOLOGICAL SCIENCES, vol. 103, 2007, pages 236P, XP022671772, ISSN: 1347-8613
- NAKAMOTO, KAZUO ET AL.: "Antinociceptive effects of docosahexaenoic acid against various pain stimuli in mice", BIOLOGICAL AND PHARMACEUTICAL BULLETIN, vol. 33, no. 6, 2010, pages 1070 - 1072, XP008136227, ISSN: 0918-6158
- NOBRE, M. E. P. ET AL.: "Eicosapentaenoic acid and docosahexaenoic acid exert anti-inflammtory and antinociceptive effects in rodents at low doses", NUTRITION RESEARCH, vol. 33, no. 5, 2013, pages 422 - 433, XP028563943, ISSN: 0271-5317

## Description

### [Technical Field]

The present invention relates to a composition for use in a method of preventing or improving nociceptive pain, which contains L-serine and n-3 fatty acid as active ingredients.

### [Background Art]

Pain is classified into "neuropathic", "nociceptive" and "psychogenic", and these three act independently or compositely to cause a pain. Pain includes acute pain and chronic pain, and acute pain that follows damage on the body tissues functions as an alert of body tissue damage, and leads to the repair of the body tissue in a given period. On the other hand, a chronic pain does not act as a defensive biological function, and is considered to last for several months to several years or longer (non-patent document 1).

According to a major research relating to pain, about 23% of the Japanese adults have a chronic pain, and 70% thereof fail to appropriately alleviate the pain (non-patent document 2). Since psychological anxiety and melancholiness associated with pain have a high impact on the limitation of activity, removal of pain improves quality of life.

Peripheral neuropathy has conventionally been reported to be prevented or improved by a composition containing serine and n-3 fatty acid (patent document 1). However, it has not been reported that nociceptive pain with a completely different mechanism of development from peripheral neuropathy can be prevented or improved by a composition containing serine and n-3 fatty acid. As a therapeutic drug for nociceptive pain, non-steroidal antiinflammatory drugs (NSAIDs), steroidal antiinflammatory agents or the like are used (non-patent document 3). However, pain often relapses when the use is discontinued, even though analgesic effects are shown during use. On the other hand, long-term use of these medicaments is associated with risk due to side effects and drug resistance, and existing therapeutic drugs are not sufficient in terms of both efficacy and safety. Patent documents 2-3 and non-patent documents 4-7 disclose further compositions.

### [Document List]

patent document 1: WO 2015/163316; patent document 2: WO 2016/152054 A1; patent document 3: WO 2015/142702 A1
non-patent document 1: Neurological Therapeutics, vol. 27, No. 4, p591-622 (2010); non-patent document 2: Clinical Orthopaedic Surgery, vol. 47, No. 2, p127-134 (2012); non-patent document 3: Journal of Allied Health Sciences, vol.8, No.1, p50-63 (2017); non-patent document 4: Nutrition Research, vol. 33, No. 5, p422-433 (2013); non-patent document 5: European Journal of Pharmacology, vol. 565, No.1-3, p89-97 (2007); non-patent document 6; Neuroscience, vol. 174, p190-199 (2011); non-patent document 7: European Spine Journal, vol. 24, No. 3, p503-512 (2014)

### [Summary of Invention]

### [Technical Problem]

The problem to be solved by the present invention is to provide a composition having a prophylactic or improvement effect on nociceptive pain.

### [Solution to Problem]

The present inventors have conducted intensive studies in an attempt to solve the aforementioned problems and found that nociceptive pain can be prevented or improved by using serine and n-3 fatty acid in combination. They have conducted further studies based on such finding, and completed the present invention.

Therefore, the present invention is as described below. Any references in the description to methods of treatment refer to the compounds, pharmaceutical compositions and medicaments of the present invention for use in a method for treatment of the human or animal body by therapy.

A composition for use in a method of preventing or improving nociceptive pain, which composition comprises an amino acid comprising L-serine, and a lipid comprising n-3 fatty acid.

In some examples, the n-3 fatty acid comprises one or more kinds selected from the group consisting of eicosapentaenoic acid, docosapentaenoic acid and docosahexaenoic acid.

In some examples, the amount of L-serine is not less than 50 wt% relative to the total amount of amino acid.

In some examples, the composition is (1) substantially free of threonine, or has (2) a weight ratio of L-serine relative to threonine of not less than 2.60.

In some examples, the amount of n-3 fatty acid is not less than 20 wt% relative to the total amount of the lipid.

In some examples, the composition has a unit package form per single intake, and comprises not less than 0.1 g of L-serine in one unit, and not less than 0.03 g of n-3 fatty acid in one unit.

In some examples, the nociceptive pain is a nociceptive pain in a joint or limb.

In some examples, the composition is a medicament or food. in some examples, the composition is for use in a method of preventing or improving a decrease in QOL related with nociceptive pain.

Also disclosed is a method for preventing or improving nociceptive pain, comprising administering an effective amount of a composition comprising an amino acid comprising serine, and a lipid comprising n-3 fatty acid to a subject in need thereof.
acid comprising serine, and a lipid comprising n-3 fatty acid, which is for use for preventing or improving nociceptive pain. In some examples of the disclosed method, composition, or use, the n-3 fatty acid comprises one or more kinds selected from the group consisting of eicosapentaenoic acid, docosapentaenoic acid and docosahexaenoic acid.

In some examples of the disclosed method, composition, or use, the amount of serine contained in the aforementioned composition is not less than 50 wt% relative to the total amount of amino acid.

In some examples of the disclosed method, composition, or use, (1) the aforementioned composition is substantially free of threonine, or (2) has a weight ratio of serine of not less than 2.60 relative to threonine in the aforementioned composition. In some examples of the disclosed method, composition, or use, the amount of n-3 fatty acid contained in the aforementioned composition is not less than 20 wt% relative to the total amount of the lipid.

In some examples of the disclosed method, composition, or use, the aforementioned composition has a unit package form per single intake, and comprises not less than 0.1 g of serine in one unit, and not less than 0.03 g of n-3 fatty acid in one unit.
or use, the nociceptive pain is a nociceptive pain in a joint or limb.

In some examples of the disclosed method, composition, or use, the aforementioned composition is a medicament or food.

Also disclosed is a method for preventing or improving a decrease in QOL related with nociceptive pain, comprising administering an effective amount of a composition comprising an amino acid comprising serine, and a lipid comprising n-3 fatty acid to a subject in need thereof.

Also disclosed is a composition comprising an amino acid comprising serine, and a lipid comprising n-3 fatty acid, which is for use for preventing or improving a decrease in QOL related with nociceptive pain.

Also disclosed is use of a composition comprising an amino acid comprising serine, and a lipid comprising n-3 fatty acid for producing an agent for preventing or improving nociceptive pain. Also disclosed is use of a composition comprising an amino acid comprising serine, and a lipid comprising n-3 fatty acid for the production of a prophylactic or improving agent for decrease in QOL related with nociceptive pain.

### [Advantageous Effects of Invention]

According to the present invention, a composition for use in a method of preventing or improving nociceptive pain according to claim 1 can be provided.

According to the present invention, a composition for use in a method of preventing or improving a decrease in QOL (Quality of Life) related with nociceptive pain according to claim 9 can be provided.

Furthermore, since the composition contains L-serine and n-3 fatty acid with established safety as active ingredients, it can be ingested safely for a long term.

### [Description of Embodiments]

The composition for preventing or improving nociceptive pain of the present invention (hereinafter to be also simply referred to as "the composition of the present invention") is mainly characterized in that it contains an amino acid comprising L-serine, and a lipid comprising n-3 fatty acid.

### [amino acid containing L-serine]

An amino acid used in the present invention essentially contains L-serine. The form of L-serine is not particularly limited, and may be a free form, as well as a form that can produce free L-serine by enzymatic reaction, or hydrolysis, for example in vivo (e.g., amino acid constituting peptide, or amino acid constituting protein). When free L-serine is used, it may be a salt or any form of the solvated thereof, or a mixture of these.

Examples of the salt of L-serine include acid addition salt, salt with base for example, and a physiologically acceptable salt is preferable.

Examples of the acid that forms a physiologically acceptable salt of L-serine include inorganic acids such as hydrogen chloride, hydrogen bromide, sulfuric acid, or phosphoric acid; and organic acids such as acetic acid, lactic acid, citric acid, tartaric acid, maleic acid, fumaric acid, or monomethyl sulfuric acid.

Examples of the base that forms a physiologically acceptable salt of L-serine include inorganic bases such as hydroxide or carbonate of metal (e.g., sodium, potassium, or calcium), or ammonia ; and organic bases such as ethylenediamine, propylenediamine, ethanolamine, monoalkylethanolamine, dialkylethanolamine, diethanolamine, or triethanolamine.

A salt of only one kind of these salts or two or more kinds of salts in combination may be used.

The production method of serine is not particularly limited, and a method known per se (e.g., protein hydrolysis method, chemical synthesis method, enzyme method, or fermentation method) can be used for production. A commercially available product may also be used. Serine can also be obtained by enzymatic hydrolysis of an animal or plant-derived natural protein having an amino acid sequence containing the serine residue.

The amino acid contained in the composition of the present invention may be composed of L-serine alone. The composition of the present invention may contain, in addition to L-serine, an amino acid other than L-serine (e.g., threonine, or glycine). The form of these amino acids is not particularly limited, and may be a free form and a salt thereof, or a form of peptide wherein two or more amino acids are linked by a peptide bond. Also, it may have a form of a protein, and examples of the protein include animal-derived protein (e.g., casein, acid casein, casein sodium, casein calcium, whey protein, milk serum whey protein, fish meat protein, egg protein, and these hydrolysate) and plant-derived protein (e.g., soybean protein, wheat protein, corn protein, and these hydrolysate) and the like. The amino acid may be any of L-form, D-form and DL-form.

The amount of serine in the composition of the present invention is preferably not less than 50 wt%, more preferably not less than 70 wt%, particularly preferably not less than 90 wt%, relative to the total amount of amino acid. The amount of serine in the composition of the present invention is calculated as a total weight of serine in any form contained in the composition of the present invention. When the form of serine is other than a free form, for example, salt or amino acid constituting peptide and protein, the weight of the serine is converted to the weight of a free form. The "total amount of amino acid" is calculated as a total weight of amino acid in any form (e.g., free amino acid and a salt thereof, peptide, or protein) contained in the composition of the present invention. When the form of amino acid is other than a free form, for example, salt or peptide and protein, the weight of the amino acid is converted to the weight of a free form.

The upper limit of the amount of serine in the composition of the present invention is not particularly set.

When the amino acid used in the present invention contains threonine, the weight ratio of serine to threonine (serine/threonine) is preferably not less than a particular value. When the weight ratio of serine to threonine (serine/threonine) is not less than a particular value, the composition of the present invention can exhibit a desired effect (e.g., nociceptive pain relieving effect) sufficiently.

Specifically, the weight ratio of serine to threonine (serine/threonine) is preferably not less than 2.60, more preferably not less than 5.20, particularly preferably not less than 10.40.

The weight ratio of serine to threonine (serine/threonine) is a value obtained by dividing the amount of serine in the composition of the present invention by the amount of threonine in the present invention. The amount of threonine in the composition of the present invention is calculated as a total weight of threonine in any form contained in the composition of the present invention. When the form of threonine is other than a free form, for example, salt or amino acid constituting peptide and protein, the weight of the threonine is converted to the weight of a free form.

The weight ratio of serine and threonine (serine/threonine) contained in 100 g of a food edible part in a natural food material is shown in the following Table 1 (calculated based on "STANDARD TABLES OF FOOD COMPOSITION IN JAPAN: AMINO ACID COMPOSITION OF FOODS 2010" (the Subdivision on Resources, the Council for Science and Technology, Ministry of Education, Culture, Sports, Science and Technology, Japan)). As shown in Table 1, a food having a weight ratio of serine to threonine (serine/threonine) of not less than 2.60 cannot be realized with a natural food material. Such food requires addition of, for example, serine in a free form to a natural food material.

**[Table 1]**

| | serine/threonine |
|---|---|
| raw milk (Holstein) | 1.15 |
| rice (milled rice) | 1.48 |
| sweet potato (tuberous root, raw) | 0.97 |
| soybean (dried) | 1.33 |
| onions (bulb, raw) | 1.50 |
| apples (raw) | 1.20 |
| Shiitake mushroom (raw) | 0.92 |
| Wakame (salted) | 0.89 |
| jack mackerel (raw) | 0.85 |
| pacific saury (raw) | 0.90 |
| Shiba shrimp (raw) | 0.94 |
| beef, comminuted meat (raw) | 0.85 |
| pork, comminuted meat (raw) | 0.89 |
| chicken, leg (with skin, raw) | 0.92 |
| egg of hen, whole egg (raw) | 1.57 |

The upper limit of the weight ratio of serine to threonine (serine/threonine) is not particularly limited, a smaller amount of threonine is more preferable, and the composition of the present invention is most preferably substantially free of threonine. The weight ratio of threonine to serine (threonine/serine, i.e., value obtained by dividing the amount of threonine in the composition of the present invention by the amount of serine in the present invention) is preferably not more than 0.38, more preferably not more than 0.20, particularly preferably not more than 0.10, most preferably 0. Being "substantially free of" threonine here means either (a) completely free of threonine, or (b) containing threonine in an amount not influential on the effect of the composition of the present invention (e.g., not more than 1 wt%, preferably not more than 0.1 wt%, relative to the total amount of amino acid).

In addition, since even threonine taken from meal for example (e.g., threonine contained in protein) may influence the effect of the composition of the present invention, ingestion of the composition of the present invention and the ingestion of threonine (e.g., meal) are preferably at least 1 hour (preferably not less than 2 hours) apart.

The total amount amino acid contained in the composition of the present invention is generally 1 - 90 wt%, preferably 5 - 85 wt%, relative to the whole composition.

### [lipid containing n-3 fatty acid]

The lipid used in the composition of the present invention essentially contains n-3 fatty acid. The "n-3 fatty acid" in the present specification means an unsaturated fatty acid having a double bond at the third carbon atom from the terminal methyl group of the hydrocarbon chain, and specific examples thereof include eicosapentaenoic acid, docosahexaenoic acid, α-linolenic acid, and docosapentaenoic acid. The n-3 fatty acid used in the composition of the present invention is preferably eicosapentaenoic acid, docosahexaenoic acid or docosapentaenoic acid, particularly preferably eicosapentaenoic acid. These n-3 fatty acids may be used alone, or two or more kinds thereof may be used in combination.

The n-3 fatty acid is abundantly contained in fats and oils such as fish oil, Japanese basil oil, or flaxseed oil, and n-3 fatty acid extracted and purified from these fats and oils can be used. In addition, n-3 fatty acid produced by a method known per se (e.g., chemical synthesis method, or fermentation method) can also be used. A commercially available product marketed for foods can also be used. In addition, fats and oils abundantly containing n-3 fatty acid can also be used directly.

The lipid contained in the composition of the present invention may contain n-3 fatty acid as well as other lipids. Examples of the lipid include edible vegetable oils such as cottonseed oil, sunflower oil, peanuts oil, rapeseed oil, soybean oil, safflower oil, olive oil, rice oil, corn oil, sesame oil, or cacao butter; edible animal oils such as beef tallow, lard, fish oil, butter, or butter oil; processed fat and oils such as shortening; medium chain fatty acid oils such as coconut oil, palm oil, or palm kernel oil. These lipids may be used alone, or two or more kinds thereof may be used in combination.

The amount of n-3 fatty acid contained in the composition of the present invention is preferably not less than 20 wt%, more preferably not less than 25 wt%, particularly preferably not less than 30 wt%, relative to the total amount of lipid contained in the composition of the present invention.

The upper limit of the amount of n-3 fatty acid is not particularly limited.

The amount of n-3 fatty acid contained in the composition of the present invention is generally 10 - 200 wt%, preferably 20 - 100 wt%, relative to the total amount of serine contained in the composition of the present invention, and more preferably 20 - 60 wt% to reduce oil ingestion as much as possible.

When n-3 fatty acid contains eicosapentaenoic acid, the content of eicosapentaenoic acid relative to the total amount of n-3 fatty acid is generally 5 - 100 wt%. To suppress oil ingestion as much as possible, it is preferably 10 - 100 wt%, more preferably 50 - 100 wt%.

The total amount of lipid contained in the composition of the present invention is generally 1 - 90 wt%, preferably 5 - 85 wt%, relative to the whole composition.

The composition of the present invention can be provided as a medicament, or food, for example. When provided as a medicament, the subject of administration is, for example, a mammal (e.g., human, mouse, rat, hamster, rabbit, cat, dog, bovine, sheep, or monkey), preferably human. The "food" in the present specification is a concept widely encompassing those that can be ingested orally (excluding medicament), and includes drinks, seasoning, or food additive for example.

When the composition of the present invention is provided as a medicament, the dosage form thereof is not particularly limited, and may be any of oral medicament and parenteral medicament. Examples of the oral medicament include tablet, granule, powder, capsule (including soft capsule), elixir, syrup, microcapsule, drink, emulsion, or suspension, and examples of the parenteral medicament include skin external preparation (e.g., ointment, cream, gel, liquid, lotion, facial mask, or bathing powder), or injection. The composition can also be used in combination with analgesic (opioid central analgesic; steroid; non-steroidal anti-inflammatory analgesic, for example) commercially available at present.

When the composition of the present invention is provided as a food, the form thereof is not particularly limited, and may be, for example, powdered product, granular product, capsular product, tablet-like product, liquid product (e.g., drinks), jelly-like drink, jelly-like product (e.g., jelly), gum-like product, sheet-like product, or solid product (e.g., snack stick, cookie).

The composition of the present invention can also be provided as a food prescribed by the Ordinance of the Ministry of Health, Labour and Welfare, such as food with health claims. The food with health claims also includes a food with an indication that it is used for particular applications (e.g., food for specified health uses, or food with nutrient function claims). In addition, the composition of the present invention can also be utilized as a food supplement. The food supplement here refers to those that are ingested for the purpose of supplementing nutrition besides those ingested as foods, and examples thereof include nutritional supplements and supplements (e.g., dietary supplements).

L-serine and n-3 fatty acid, which are active ingredients of the composition of the present invention, can be each singly or combinedly contained in plural (two or more) compositions. The plural compositions may be, for example, a combination of two or more medicaments, a combination of two or more foods, or a combination of one or more medicaments and one or more foods,.

When L-serine and n-3 fatty acid are contained in plural compositions, the amount of L-serine relative to the total amount of amino acid, the amount of n-3 fatty acid relative to the total amount of lipid, or the content of eicosapentaenoic acid relative to the total amount of n-3 fatty acid for example are calculated from the total amount of each component contained in plural compositions.

The composition of the present invention may contain carriers conventionally used in the field of medicament or food as necessary, as long as the object of the present invention is not impaired.

When the composition of the present invention is an oral medicament, or food, for example, examples of the carrier that may be contained include binders such as tragacanth, gum arabic, cornstarch, gelatin, or high molecular weight polyvinylpyrrolidone; excipients such as cellulose and a derivative thereof (e.g., microcrystalline cellulose, crystalline cellulose, or hydroxypropyl cellulose); swelling agents such as cornstarch, pregelatinized starch, alginic acid, or dextrin; lubricants such as magnesium stearate; flowability improving agents such as particle silicon dioxide, or methyl cellulose; lubricants such as glycerin fatty acid ester, talc, or polyethylene glycol 6000; thickeners such as sodium carboxymethyl cellulose, carboxyvinyl polymer, xanthan gum, or gelatin; sweetening agents such as sucrose, lactose, or aspartame; flavors such as peppermint flavor, vanilla flavor, cherry flavor, or orange flavor; emulsifiers such as monoglyceride, polyglycerol fatty acid ester, sucrose fatty acid ester, lecithin (e.g., soybean lecithin), polyoxyethylene hydrogenated castor oil, polyoxyethylene monostearic acid ester, or beeswax; pH adjusters such as citric acid, sodium citrate, acetic acid, sodium acetate, or sodium hydroxide; thickeners such as sodium carboxymethyl cellulose, carboxyvinyl polymer, xanthan gum, or gelatin; corrigents such as aspartame, licorice extract, or saccharin; antioxidants such as erythorbic acid, butylated hydroxyanisole, or propyl gallate; preservatives such as sodium benzoate, sodium edetate, sorbic acid, sodium sorbate, methyl paraoxybenzoate, or butyl paraoxybenzoate; colorants such as red iron oxide, yellow iron oxide, black iron oxide, carmine, Food Color Blue No. 1, Food Color Yellow No. 4, or Food Color Red No. 2; fats and oils such as safflower oil; antioxidants such as vitamin C, vitamin A, vitamin E, various polyphenol, hydroxytyrosol, or antioxidant amino acid; coating agents such as shellac, sugar, hydroxypropyl methylcellulose phthalate, or polyacetin; preservatives such as methylparaben, or propylparaben; various vitamins; various amino acids.

When the composition of the present invention is a parenteral medicament for example, examples of the carrier that may be contained include higher fatty acid esters such as petrolatum, liquid paraffin, isopropyl myristate, or octyldodecyl myristate; higher alcohols such as squalane, lanolin, or cetanol; grease bases such as silicone oil, or oil from plant or animal; lower alcohols such as ethanol; polyhydric alcohols such as polyethylene glycol, or propylene glycol; emulsifiers or emulsion stabilizers such as α-monoglyceryl ether, lecithin, sorbitan fatty acid ester, dextrin fatty acid ester, fatty acid monoglyceride, fatty acid metal salt, or magnesium sulfate; aromatic; preservative; dye; thickener; antioxidant; UV defense agent; wound therapeutic agent; anti-inflammatory agent; humectant; water.

The composition of the present invention can be formulated as a unit package form per single intake. In the present specification, the "unit package form per single intake" means a package form of one or more units with single intake as one unit. For the package, a packaging material and a packaging method, a filling method (e.g., divided package, or stick package) generally used for a package of medicament, or food, for example can be used.

In the present specification, the "single intake" is, for example, the amount of the composition to be administered at one time when the composition of the present invention is a medicament. When the composition of the present invention is a food, it is the amount of the composition ingested in one meal. The single intake can be appropriately controlled according to the age, body weight, sex for exampleof the subject who ingests.

A single intake of the composition of the present invention can be appropriately set according to the form, dosage form, or ingestion subject for example of the composition and is not particularly limited. For a general adult (body weight 60 kg), 0.2 - 12.0 g is preferable, 0.3 - 10.0 g is more preferable, and 0.5 - 8.0 g is particularly preferable. When the single intake of the composition of the present invention is within the above-mentioned range, it does not influence general meals much, and a sustained ingestion is expected.

The composition of the present invention in a unit package form per single intake preferably contains not less than 0.1 g (more preferably not less than 0.2 g, particularly preferably not less than 0.3 g) of serine in one unit. The content of serine in this case is preferably not more than 10.0 g (more preferably not more than 8.0 g, particularly preferably not more than 5.0 g) in one unit. When the content is less than 0.1 g in one unit, serine is utilized as a protein constituting component or energy source in the body and a desired effect tends to be unexpected. When the content exceeds 10.0 g in one unit, a large amount of a single amino acid is ingested, which is not very preferable from the aspect of amino acid balance.

When the composition of the present invention is in a unit package form per single intake, the content of threonine is preferably not more than 0.15 g (more preferably not more than 0.1 g, further preferably not more than 0.05 g, particularly preferably not more than 0.025 g), in one unit.

When the composition of the present invention is in a unit package form per single intake, n-3 fatty acid is preferably contained at not less than 0.03 g (more preferably not less than 0.04 g, particularly preferably not less than 0.05 g) in one unit. In this case, the content of n-3 fatty acid is preferably not more than 6.00 g (more preferably not more than 4.50 g, particularly preferably not more than 3.00 g) in one unit. When the content is less than 0.03 g in one unit, a desired effect tends to be not clearly expected. A content exceeding 6.00 g in one unit is not very preferable from the aspect of flavor.

When the composition of the present invention is a unit package form per single intake and n-3 fatty acid contains eicosapentaenoic acid, it contains eicosapentaenoic acid at preferably 20 - 4000 mg, more preferably 30 - 3000 mg, particularly preferably 40 - 2000 mg, in one unit.

While the daily ingestion of serine can be appropriately set according to the age, sex, body weight, meal state for example of the subject who ingests, it is generally 0.1 - 10.0 g, preferably 0.2 - 8.0 g, more preferably 0.3 - 5.0 g, for an ordinary adult (body weight 60 kg).

In addition, ingestion of not less than 2 g of n-3 fatty acid per day is recommended in Japan for an ordinary adult (body weight 60 kg) based on the lipid ingestion state in the past.

Ingestion of not less than 1 g of eicosapentaenoic acid concurrently with docosahexaenoic acid per day is recommended in Japan for an ordinary adult (body weight 60 kg) based on the lipid ingestion state in the past.

The composition of the present invention is preferably ingested once to several times per day (preferably 1 - 3 times per day), so that the daily ingestion of serine, n-3 fatty acid and eicosapentaenoic acid will each fall within the above-mentioned ranges.

The ingestion period of the composition of the present invention (period of from the date of first ingestion to the date of final ingestion) is not particularly limited, and it is generally not less than 3 days, preferably not less than 1 week, more preferably not less than 4 weeks, particularly preferably not less than 8 weeks. The upper limit of the ingestion period is not particularly set.

For application of the composition of the present invention to an animal other than human, the above-mentioned single intake, content in one unit, daily intake for example can be appropriately increased or reduced based on the above-mentioned amount for an ordinary adult, and further considering the body weight or size of the animal, or the condition, sensitivity for example of the animal at the time of administration.

For application of the composition of the present invention to a human, the dose of each component (e.g. L-serine) in the composition of the present invention can be determined based on the experiment results of each animal other than human.

The composition of the present invention can be produced by a method known per se in the technical field of preparation formulation (e.g., the method described in the Japanese Pharmacopoeia, 16th Edition, the method described in WO 2015/163316, or the method described in US-B-2017/0035721 for example).

The composition of the present invention is useful for preventing or improving nociceptive pain. The "nociceptive pain" in the present specification refers to pain caused by damage or possible damage to tissues by noxious stimulus or inflammation.

In the present invention, pain can be classified by a method known per se or a method analogous thereto. For example, pain can be classified into nociceptive (score: 0-12 points), neuropathic (score: 19-38 points), and both (score: 13-18 points) using PainDETECT Questionnaire developed by Freynhagen et al. (Curr Med Res Opin. 2006; 22(10); 1911-20).

The nociceptive pain that can be prevented or improved by the composition of the present invention may be acute or chronic, and the site of pain is not particularly limited. For example, joints of neck (e.g., facet joint of cervical spine,), waist (e.g., lumbar facet joint, or sacroiliac joint,), backbone (e.g., spinal facet joint), knee (e.g., knee joint), shoulder (e.g., scapula, or shoulder joint,); muscles such as skeletal muscle, or smooth muscle; internal organs such as stomach, intestine, liver, or kidney; limbs such as finger, palm of hand, back of hand, toe, sole of foot, or instep of foot; skin (including mucosa); bone; or tooth can be mentioned. The cause of nociceptive pain (injury, or disease for example) is not particularly limited and, for example, bruise, bone fracture, burn, osteoarthritis, rheumatoid arthritis, stiff neck, or tenosynovitis can be mentioned. The nociceptive pain that can be prevented or improved by the composition of the present invention may be accompanied by stiffness, swelling and other symptoms.

In the present specification, the "prevention" of nociceptive pain means prevention (including prevention of recurrence) of the perception of pain in individuals who have not perceived nociceptive pain. In addition, the "prevention" of nociceptive pain also includes prevention (including prevention of recurrence) of the manifestation of symptoms related with nociceptive pain (e.g., stiffness, or swelling,) in individuals who have not exhibited the symptoms.

In the present specification, the "improvement" of nociceptive pain means reducing the intensity of pain, or preventing or delaying an increase in the intensity of pain in individuals who are perceiving nociceptive pain. In addition, the "improvement" of nociceptive pain also includes reducing the symptoms related with nociceptive pain (e.g., stiffness, or swelling) or preventing or delaying the exacerbation of the symptoms in individuals exhibiting the symptoms.

The composition of the present invention is preferably used for preventing or improving nociceptive pain in a joint or limb, more preferably used for preventing or improving nociceptive pain in a joint, and particularly preferably used for preventing or improving nociceptive pain in the waist or knee.

As shown in the below-mentioned Example, the composition of the present invention is also useful for preventing or improving a decrease in QOL related with nociceptive pain, and can be provided as a composition for preventing or improving a decrease in QOL related with nociceptive pain.

A specific embodiment of a decrease in QOL related with nociceptive pain is, for example, that nociceptive pain makes it difficult to perform at least one action selected from the group consisting of physically intense activities (e.g., exercise, or labor,), carrying of objects (e.g., carrying of heavy object), walking, stepping stairs, standing up, crouching, bending, and kneeling. The composition of the present invention is useful for improving or preventing, for example, difficulty in performing at least one action selected from the group consisting of physically intense activities, carrying of objects, walking, stepping stairs, standing up, crouching, bending, and kneeling.

In the present specification, the "prevention" of a decrease in QOL related with nociceptive pain means preventing a decrease in QOL related with the occurrence of nociceptive pain by preventing the perception of nociceptive pain in individuals who have not perceived nociceptive pain.

In the present specification, the "improvement" of a decrease in QOL related with nociceptive pain means improving QOL, maintaining QOL, or reducing or delaying a decrease in QOL in individuals who are perceiving nociceptive pain.

The preventive or improving effect of nociceptive pain and the preventive or improving effect of a decrease in QOL related with nociceptive pain according to the present invention may be evaluated by a method known per se, and the evaluation is not particularly limited. For example, the evaluation can be performed by questionnaire survey for example using questionnaires. The questionnaire survey can be performed by a method known per se. For example, the score of each item of the questionnaires is measured before ingesting the test sample and after ingesting the test sample continuously for a certain period (e.g., 1 week to 8 weeks), and when the score after ingestion of the test sample significantly increased or tended to increase as compared with that before ingestion, the nociceptive pain can be evaluated to have been prevented or improved for example. Examples of the questionnaire that can be used for the evaluation of the effects of the present invention include Knee Injury and Osteoarthritis Outcome Score (KOOS) and its Japanese version (J-KOOS), Japanese Orthopaedic Association Back Pain Evaluation Questionnaire (JOABPEQ), SF-36 (registered trade mark) (Medical outcomes study 36-Item Short-Form Health Survey), or questionnaires similar to these.

When conducting a questionnaire survey using KOOS, J-KOOS, or a questionnaire similar thereto, the composition of the present invention can significantly improve, or can achieve an improving tendency of, the score of one or more items selected from the group consisting of items relating to "stiffness; pain", items relating to "function" and items relating to "quality of life".

When conducting a questionnaire survey using JOABPEQ or a questionnaire similar thereto, the score of one or more items selected from the group consisting of items relating to "strength of low back pain", items relating to "physical function", items relating to "walking/movement", items relating to "daily role/social life", and items relating to "vitality/mentality" can be significantly improved or show an improving tendency.

When conducting a questionnaire survey using SF-36 or a questionnaire similar thereto, the composition of the present invention can significantly improve, or can achieve an improving tendency of, the score of one or more items selected from the group consisting of items relating to "physical function", items relating to "bodily pain", items relating to "vitality" and items relating to "role emotional".

The present disclosure also provides a method for preventing or improving nociceptive pain, including administering an effective amount of the composition of the present invention to a subject in need thereof. The present disclosure also provides a method for preventing or improving nociceptive pain, which includes administering an effective amount of serine and n-3 fatty acid to a subject in need thereof.

The present disclosure also provides a method for preventing or improving a decrease in QOL related with nociceptive pain, which includes administering an effective amount of the composition of the present invention to a subject in need thereof. The present disclosure also provides a method for preventing or improving a decrease in QOL related with nociceptive pain, which includes administering an effective amount of serine and n-3 fatty acid to a subject in need thereof.

These methods may exclude a medical practice. The "medical practice" here means an act of treating, operating on or diagnosing human, which is performed by physicians or dentists, or under instruction and supervision of physicians or dentists.

The present invention is explained in more detail in the following Examples; however, the present invention is not limited by these examples in any way. In the following Examples, "%" means "wt%", unless otherwise specified.

### [Example]

### (Selection of subjects)

A questionnaire survey using PainDETECT Questionnaire was conducted on 57 adult men and women with pain, numbness, and discomfort in joint, limb, and the like, and 14 subjects (7 females and 7 males) whose scores were within the range of 0 to 12 were selected as subjects having nociceptive pain. The age, height and body weight of the test subjects were respectively 54.1±11.8 years old, 163.6±7.4 cm and 67.0±11.4 kg (each average±standard deviation).

### (Production of test sample)

As a test sample, the following capsule was produced by a conventional method.
shape: OVAL-6 soft capsule (brown)
capsule specification: sodium phosphate-free vegetable-coated soft capsule (manufactured by Catalent Japan K.K., Vegicaps (registered trade mark))
composition: containing components listed in the following Table 2 in one capsule (total amount: 344 mg). In the Table, "EPA-28" is refined fish oil containing 28% of eicosapentaenoic acid and also containing 12% of docosahexaenoic acid (manufactured by Nippon Suisan Kaisha, Ltd.).

**[Table 2]**

| component | content (mg) |
|---|---|
| L-serine | 156.24 |
| EPA-28 | 156.24 |
| safflower oil | 8.50 |
| beeswax | 22.02 |
| soybean lecithin | 1.00 |

### (Test method)

Each test subject ingested the test sample once per day for 8 weeks. The ingestion amount of the test sample was 4 capsules once per day. The ingestion method was oral ingestion with water before bedtime at least 2 hr after dinner. When ingestion before bedtime is difficult, the test sample was orally ingested after 2 hr or more from the meal, and no meal was taken for at least 2 hr after the ingestion.

A questionnaire survey regarding knee condition, waist condition and health-related QOL was conducted for each subject 1 week before the start of ingestion of the test sample, the date of start of ingestion, 1 week, 4 weeks and 8 weeks after the start of ingestion. In the questionnaire survey on the knee condition, a questionnaire (J-KOOS improved version) modified in 25 items regarding "stiffness; pain", "function", and "quality of life" based on all 42 items of J-KOOS was used. In the questionnaire survey on the waist condition, a questionnaire in which the way JOABPEQ is scored was changed and the questions about the degree of pain and numbness were removed (JOABPEQ improved version) was used. In the questionnaire survey on the health-related quality of life, a questionnaire in which the way SF-36 is scored was changed (SF-36 improved version) was used.

### (Evaluation)

The score of each questionnaire was calculated 1 week before the start of ingestion of the test sample, the date of start of ingestion, 1 week, 4 weeks and 8 weeks after the start of ingestion, the average of the score one week before the start of ingestion of the test sample and the score on the date of start of the ingestion was set as the "baseline", and the scores 1 week, 4 weeks and 8 weeks after the start of ingestion were each compared with the baseline.

The results are shown in the following Table 3 (results of questionnaire survey using J-KOOS improved version), Table 4 (results of questionnaire survey using JOABPEQ improved version) and Table 5 (results of questionnaire survey using SF-36 improved version).

**[Table 3]**

| Results questionnaire survey using J-KOOS improved version | | | | |
|---|---|---|---|---|
| | baseline (average of one week before start of ingestion and date of start of ingestion) | one week after start of ingestion | 4 weeks after start of ingestion | 8 weeks after start of ingestion |
| total score of all items | 112.6 ± 11.2 | 116.4 ± 8.3 ** | 117.7 ± 7.9 ** | 118.6 ± 8.7 ** |
| total score of items relating to "stiffness; pain" | 29.4 ± 4.0 | 30.8 ± 3.5 ** | 31.6 ± 3.3 *** | 32.4 ± 3.0 *** |
| total score of items relating to "function" | 69.1 ± 6.6 | 70.9 ± 4.6 * | 71.4 ± 4.6 * | 71.5 ± 5.7 * |
| total score of items relating to "quality of life" | 14.1 ± 1.0 | 14.7 ± 0.6 * | 14.7 ± 0.5 * | 14.8 ± 0.4 * |

| | | | | |
|---|---|---|---|---|
| mean ± SD *: p<0.05, **:p<0.01, ***:p<0.001 (paired t-test) | | | | |

As is clear from the results shown in Table 3, the total score of all items significantly increased from 1 week to 8 weeks after the start of ingestion of the test sample as compared with the baseline.

In addition, the total score of the items relating to "stiffness, pain", the total score of the items relating to "function", and the total score of the items relating to "quality of life" significantly increased from 1 week to 8 weeks after the start of ingestion of the test sample as compared to the baseline.

From these results, it was suggested that the combination of serine and n-3 fatty acid is useful for preventing and improving nociceptive pain (particularly, nociceptive pain in the knee).

**[Table 4]**

| Results questionnaire survey using JOABPEQ improved version | | | | |
|---|---|---|---|---|
| | baseline (average of one week before start of ingestion and date of start of ingestion) | one week after start of ingestion | 4 weeks after start of ingestion | 8 weeks after start of ingestion |
| total score of all items | 103.0 ± 12.3 | 110.0 ± 11.8 *** | 108.7 ± 12.1 * | 111.9 ± 9.9 *** |
| total score of items relating to "strength of low back pain" | 16.1 ± 2.6 | 16.9 ± 2.5 ^{#} | 17.0 ± 2.4 | 17.7 ± 2.6 ^{#} |
| total score of items relating to "physical function" | 26.9 ± 3.2 | 28.1 ± 3.9 * | 27.8 ± 4.1 | 28.4 ± 3.5 ** |
| total score of items relating to "walking/movement" | 21.5 ± 4.4 | 23.6 ± 3.3 ** | 22.9 ± 3.7 ^{#} | 23.4 ± 2.7 * |
| total score of items relating to "daily role/social life" | 13.3 ± 1.7 | 14.1 ± 1.5 ** | 14.1 ± 1.5 * | 14.4 ± 0.9 * |
| total score of items relating to "vitality/mentality" | 25.3 ± 2.7 | 27.3 ± 3.3 ** | 27.0 ± 2.9 * | 27.9 ± 2.9 ** |

| | | | | |
|---|---|---|---|---|
| mean ± SD #: p<0.1, *: p<0.05, **:p<0.01, ***:p<0.001 (paired t-test) | | | | |

As is clear from the results shown in Table 4, the total score of all items significantly increased from 1 week to 8 weeks after the start of ingestion of the test sample as compared with the baseline.

In addition, the total score of the items relating to "physical functions", the total score of the items relating to "walking/movement", the total score of the items relating to "daily role/social life", and the total score of the items relating to "vitality/mentality" each significantly increased from 1 week to 8 weeks after the start of ingestion of the test sample as compared to the baseline. The total score of the items relating to "strength of low back pain" tended to increase as compared to the baseline.

From these results, it was suggested that the combination of serine and n-3 fatty acid is useful for preventing and improving nociceptive pain (particularly, nociceptive pain in the waist).

**[Table 5]**

| Results questionnaire survey using SF-36 improved version | | | | |
|---|---|---|---|---|
| | baseline (average of one week before start of ingestion and date of start of ingestion) | one week after start of ingestion | 4 weeks after start of ingestion | 8 weeks after start of ingestion |
| total score of all items | 145.7 ± 14.9 | 151.9 ± 12.9 ** | 152.6 ± 12.3 * | 153.2 ± 10.6 * |
| total score of items relating to "physical function" | 41.8 ± 6.0 | 43.9 ± 4.9 * | 44.0 ± 5.9 * | 45.2 ± 4.5 ** |
| total score of items relating to "bodily pain" | 7.9 ± 0.9 | 8.9 ± 1.2 *** | 8.6 ± 1.3^{#} | 8.8 ± 1.4 * |
| total score of items relating to "vitality" | 13.5 ± 2.3 | 14.1 ± 2.0 ^{#} | 14.5 ± 2.0 * | 14.8 ± 2.0 ** |
| total score of items relating to "role emotional" | 13.7 ± 1.4 | 14.4 ± 1.3 ^{#} | 14.6 ± 0.9 ^{#} | 14.6 ± 0.9 ^{#} |

| | | | | |
|---|---|---|---|---|
| mean ± SD #: p<0.1, *: p<0.05, **:p<0.01, ***:p<0.001 (paired t-test) | | | | |

As is clear from the results shown in Table 5, the total score of all items was significantly increased from 1 week to 8 weeks after the start of ingestion of the test sample as compared with the baseline.

In addition, the total score of the items relating to "physical functions", and the total score of the items relating to "bodily paint" significantly increased from 1 week to 8 weeks after the start of ingestion of the test sample as compared to the baseline. The total score of the items relating to "vitality" significantly increased from 4 weeks to 8 weeks after the start of ingestion of the test sample as compared to the baseline. The total score of the items relating to "role emotional" tended to increase from 1 week to 8 weeks after the start of ingestion of the test sample as compared to the baseline.

From these results, it was suggested that the combination of serine and n-3 fatty acid improve QOL (physical functions, bodily pain, vitality, role emotional) by preventing or improving nociceptive pain.

### [Industrial Applicability]

According to the present invention, a composition for use in a method of preventing or improving nociceptive pain according to claim 1 can be provided.

According to the present invention, a composition for use in a method of preventing or improving a decrease in QOL related with nociceptive pain according to claim 9 can be provided.

Since the composition contains L-serine and n-3 fatty acid with established safety as active ingredients, it can be ingested safely for a long term.

This application is based on a patent application No. 2018-011964 filed in Japan (filing date: January 26, 2018).

## Claims

1. A composition for use in a method of preventing or improving nociceptive pain, which composition comprises an amino acid comprising L-serine, and a lipid comprising n-3 fatty acid.

2. The composition for use according to claim 1, wherein the n-3 fatty acid comprises one or more kinds selected from the group consisting of eicosapentaenoic acid, docosapentaenoic acid and docosahexaenoic acid.

3. The composition for use according to claim 1 or 2, wherein the amount of L-serine is not less than 50 wt% relative to the total amount of amino acid.

4. The composition for use according to any one of claims 1 to 3, wherein the composition is (1) substantially free of threonine, or has (2) a weight ratio of L-serine relative to threonine of not less than 2.60.

5. The composition for use according to any one of claims 1 to 4, wherein the amount of n-3 fatty acid is not less than 20 wt% relative to the total amount of the lipid.

6. The composition for use according to any one of claims 1 to 5, wherein the composition has a unit package form per single intake, and comprises not less than 0.1 g of L-serine in one unit, and not less than 0.03 g of n-3 fatty acid in one unit.

7. The composition for use according to any one of claims 1 to 6, wherein the nociceptive pain is a nociceptive pain in a joint or limb.

8. The composition for use according to any one of claims 1 to 7, wherein the composition is a medicament or food.

9. A composition for use according to any one of claims 1 to 8, wherein the method further prevents or improves a decrease in quality of life related with nociceptive pain of a subject.

## Patentansprüche

1. Zusammensetzung zur Verwendung in einem Verfahren zur Vorbeugung oder Verbesserung von nozizeptivem Schmerz, wobei die Zusammensetzung eine L-Serin umfassende Aminosäure und ein eine n-3-Fettsäure umfassendes Lipid enthält.

2. Zusammensetzung zur Verwendung gemäß Anspruch 1, wobei die n-3-Fettsäure eine oder mehrere Arten enthält, die aus der aus Eicosapentaensäure, Docosapentaensäure und Docosahexaensäure bestehenden Gruppe ausgewählt ist.

3. Zusammensetzung zur Verwendung gemäß Anspruch 1 oder 2, wobei die Menge an L-Serin nicht weniger als 50 Gew.-%, bezogen auf die Gesamtmenge an Aminosäure, beträgt.

4. Zusammensetzung zur Verwendung gemäß einem der Ansprüche 1 bis 3, wobei die Zusammensetzung (1) im Wesentlichen frei von Threonin ist oder (2) ein Gewichtsverhältnis von L-Serin zu Threonin von nicht weniger als 2,60 aufweist.

5. Zusammensetzung zur Verwendung gemäß einem der Ansprüche 1 bis 4, wobei die Menge an n-3-Fettsäure nicht weniger als 20 Gew.-%, bezogen auf die Gesamtmenge des Lipids, beträgt.

6. Zusammensetzung zur Verwendung gemäß einem der Ansprüche 1 bis 5, wobei die Zusammensetzung eine Einheitsverpackungsform pro Einzeleinnahme aufweist und in einer Einheit nicht weniger als 0,1 g L-Serin sowie nicht weniger als 0,03 g n-3-Fettsäure enthält.

7. Zusammensetzung zur Verwendung gemäß einem der Ansprüche 1 bis 6, wobei der nozizeptive Schmerz ein nozizeptiver Schmerz in einem Gelenk oder einer Extremität ist.

8. Zusammensetzung zur Verwendung gemäß einem der Ansprüche 1 bis 7, wobei die Zusammensetzung ein Arzneimittel oder ein Lebensmittel ist.

9. Zusammensetzung zur Verwendung gemäß einem der Ansprüche 1 bis 8, wobei das Verfahren ferner eine Abnahme der mit nozizeptivem Schmerz zusammenhängenden Lebensqualität eines Subjekts verhindert oder verbessert.

## Revendications

1. Composition pour une utilisation dans une méthode de prévention ou d'amélioration d'une douleur nociceptive, laquelle composition comprend un acide aminé comprenant de la L-sérine, et un lipide comprenant un acide gras n-3.

2. Composition pour une utilisation selon la revendication 1, dans laquelle l'acide gras n-3 comprend un ou plusieurs types choisis dans le groupe constitué par l'acide eicosapentaénoïque, l'acide docosapentaénoïque et l'acide docosahexaénoïque.

3. Composition pour une utilisation selon la revendication 1 ou 2, dans laquelle la quantité de L-sérine n'est pas inférieure à 50 % en poids par rapport à la quantité totale d'acide aminé.

4. Composition pour une utilisation selon l'une quelconque des revendications 1 à 3, laquelle composition (1) est pratiquement exempte de thréonine, ou (2) présente un rapport en poids de la L-sérine à la thréonine non inférieur à 2,60.

5. Composition pour une utilisation selon l'une quelconque des revendications 1 à 4, dans laquelle la quantité d'acide gras n-3 n'est pas inférieure à 20 % en poids par rapport à la quantité totale du lipide.

6. Composition pour une utilisation selon l'une quelconque des revendications 1 à 5, laquelle composition est sous forme d'un conditionnement unitaire destiné à une prise unique, et comprend au moins 0,1 g de L-sérine par unité, et au moins 0,03 g d'acide gras n-3 par unité.

7. Composition pour une utilisation selon l'une quelconque des revendications 1 à 6, dans laquelle la douleur nociceptive est une douleur nociceptive dans une articulation ou un membre.

8. Composition pour une utilisation selon l'une quelconque des revendications 1 à 7, laquelle composition est un médicament ou un aliment.

9. Composition pour une utilisation selon l'une quelconque des revendications 1 à 8, dans laquelle la méthode en outre empêche ou améliore une diminution de la qualité de vie associée à une douleur nociceptive d'un sujet.
